Europäisches Patentamt

European Patent Office

Office européen des brevets

(11) Numéro de publication: **0 208 615 B1**

## (12) FASCICULE DE BREVET EUROPEEN

(45) Date de publication de fascicule du brevet: **04.03.92**   (51) Int. Cl.⁵: **A61K 47/42**, A61K 39/395, A61K 47/48

(21) Numéro de dépôt: **86401482.4**

(22) Date de dépôt: **03.07.86**

(54) **Anticorps utiles comme agents de pilotage et conjugués les incorporant.**

(30) Priorité: **04.07.85 FR 8510234**

(43) Date de publication de la demande:
**14.01.87 Bulletin 87/03**

(45) Mention de la délivrance du brevet:
**04.03.92 Bulletin 92/10**

(84) Etats contractants désignés:
**AT BE CH DE FR GB IT LI LU NL SE**

(56) Documents cités:
**EP-A- 0 037 388**
**EP-A- 0 124 502**
**FR-A- 2 445 149**
**US-A- 4 376 765**

**CHEMICAL ABSTRACTS, vol. 95, no. 9, 31 août 1981, Columbus, OH (US); P.N.KULKARNI et al., pp. 27-28, no. 73122n**

**CHEMICAL ABSTRACTS, vol. 98, 1983, Columbus, OH (US); G.F.ROWLAND et al., p. 21, no. 154983h**

(73) Titulaire: **IRE-CELLTARG S.A.**
**Zone Industrielle**
**B-6220 Fleurus(BE)**

(72) Inventeur: **Schneider, Yves-Jacques Emile**
**Dennenboslaan 14**
**B-1900 Overijse(BE)**
Inventeur: **Aboud-Pirak, Esther**
**Miller Street 15**
**Rehovot(IS)**
Inventeur: **Trouet, André Benoît Léon**
**Predikherenberg 29, D.**
**B-3009 Winksele(BE)**

(74) Mandataire: **Warcoin, Jacques et al**
**Cabinet Régimbeau 26, avenue Kléber**
**F-75116 Paris(FR)**

CHEMICAL ABSTRACTS, vol. 99, 1983, Columbus, OH (US); G.MELINO et al., p. 30, no. 169139g

BIOLOGICAL ABSTRACTS, vol. 62, no. 8, 1976, E.HURWITZ et al., no. 44481

CHEMICAL ABSTRACTS, vol. 98, 1983, Columbus, OH (US); M.J.EMBLETON et al., pp. 42-43, no. 172695v

CHEMICAL ABSTRACTS, vol. 101, 1984, Columbus, OH (US); R.W.BALDWIN et al., p. 470, no. 70542f

**Description**

La présente invention concerne des anticorps utilisés comme agents de pilotage de substances à activité pharmacologique, utilisés à des fins thérapeutiques ou diagnostiques, ainsi que des conjugués actifs comportant lesdits agents de pilotage.

La technique du pilotage des substances à activité pharmacologique est connue. Elle consiste essentiellement à adjoindre à ladite substance à activité pharmacologique un anticorps présentant une affinité particulière pour une partie de l'être vivant, par exemple des cellules spécifiques, lesquelles doivent être plus particulièrement atteintes par la substance à activité pharmacologique.

En théorie, on peut donc espérer que l'agent de pilotage amènera la substance à activité pharmacologique au voisinage ou à l'intérieur des cellules à traiter ou à diagnostiquer et que, dans ces conditions, au voisinage desdites cellules la concentration en substance active sera très importante et donc permettra une meilleure activité thérapeutique ou diagnostique.

En fait, il apparaît que cette approche théorique ne conduit pas forcément à des résultats parfaitement satisfaisants.

En particulier, bien que l'on ait songé à utiliser à titre d'agents de pilotage des anticorps, les résultats qui ont pu être observés ne sont pas en rapport avec les résultats que l'on pouvait espérer.

C'est précisément l'un des objets de la présente invention que de définir les caractéristiques des anticorps ou des fragments d'anticorps pouvant être utilisés en tant qu'agents de pilotage et qui sont susceptibles de fournir des résultats prévisibles, à la différence des conjugués utilisant les techniques antérieures qui, pour l'essentiel, donnaient des résultats aléatoires suivant le type d'anticorps utilisé, ces résultats étant, en outre, très décevants par rapport à ce que l'on pouvait attendre de l'activité desdits conjugués.

C'est pourquoi la présente invention concerne à titre d'agent de pilotage de substances à activité pharmacologique ou de substances utiles à des fins diagnostiques, dans des cellules cibles de carcinomes humains un anticorps (Ac) ou un fragment d'anticorps, caractérisé en ce que :

a) il s'agit d'un anticorps monoclonal dressé contre la membrane de globules graisseux de lait humain et contre la membrane plasmique de cellules cibles de carcinomes humains,

b) les antigènes (Ag) correspondants ne sont pratiquement accessibles qu'à la surface des cellules cibles,

c) l'Ac ou le fragment d'Ac peut accéder aux cellules cibles,

d) l'Ac ou le fragment d'Ac est endocyté et à accès aux lysosomes des cellules cibles,

e) l'Ac ou le fragment d'Ac n'induit pas la disparition définitive des Ag correspondants de la surface des cellules cibles.

Dans ce qui suit, on utilisera souvent la dénomination "anticorps" pour désigner aussi bien la molécule entière d'immunoglobuline qu'un fragment d'anticorps tel que F(ab), F(ab)' ou F(ab)'$_2$, ces différents éléments étant utiles à titre d'agents de pilotage dans le cadre de la présente invention.

Le premier critère retenu pour caractériser les agents de pilotage selon la présente invention est que les antigènes correspondants ne soient pratiquement présents ou accessibles qu'à la surface des cellules cibles. Dans la majorité des cas, on souhaitera, en effet, que cet anticorps soit le plus sélectif possible ; par exemple, dans le cas du traitement des cellules tumorales que cet anticorps ne reconnaisse que les antigènes présents à la surface des cellules tumorales ; mais dans certains cas, on peut très bien envisager que cette sélectivité soit moins contraignante dans la mesure où les cellules autres que les cellules cibles qui pourraient être reconnues par les anticorps ne sont pas ou peu affectées par la substance à activité pharmacologique ou à accumulation qui est liée à l'agent de pilotage.

La sélectivité des anticorps en cause peut s'envisager à plusieurs niveaux suivant, d'une part, la nature de l'antigène visé et, d'autre part, le type d'administration que l'on envisage.

Tout d'abord, l'antigène correspondant peut être spécifique d'un organe ou d'un type de cellule précis et n'être présent qu'à la surface des cellules dudit organe ou d'un type hystologique particulier. Dans ce cas, l'anticorps reconnaitra tant les cellules normales que tumorales de cet organe ou de ce type cellulaire.

L'antigène peut être associé à une transformation cancéreuse et dans ce cas l'anticorps ne pourra reconnaître que les différents types de cellules transformées.

L'antigène peut également être spécifique d'un type de cellules tumorales et dans ce cas l'anticorps ne reconnaîtra que les cellules de ce type de tumeur.

Enfin, cet antigène peut être présent à la surface d'un type de cellules particulier d'un individu particulier, comme c'est le cas, par exemple, de l'idiotype des immunoglobulines de surface des lymphocytes a (normaux ou transformés).

Lorsque la sélectivité dépend du type d'administration de l'Ac envisagé, on peut distinguer le cas de l'administration in vivo des conjugués à des patients ; il est primordial, dans ce cas, que l'Ac puisse reconnaître toutes les cellules qui constituent la cible thérapeutique ou diagnostique, ce qui implique que l'Ag soit présent à la surface de toutes ou presque toutes les cellules tumorales ; ceci afin d'éviter, en particulier au niveau de la thérapeutique, de voir se sélectionner un type de cellule particulier qui ne serait pas touché par le conjugué. Comme cela a été dit, dans ce cas, l'Ac peut également être reconnu par d'autres cellules de l'organisme, dans la mesure où elles ne seront pas affectées par l'interaction avec la substance pilotée.

Dans d'autres cas, bien entendu, il sera indispensable d'avoir une sélectivité beaucoup plus fine, par exemple dans le cas de traitements ex vivo, comme les purges de moëlle, où il est primordial que l'Ac ne provoque pas la destruction d'autres cellules que les cellules tumorales.

La sélectivité possible des anticorps en cause, qu'ils soient monoclonaux ou polyclonaux, peut être évaluée par des techniques histo-immunochimiques qui permettent d'établir le profil de l'anticorps monoclonal et en particulier déterminer les types de cellules normales ou tumorales qu'il reconnaît et pour chaque cellule la localisation de l'interaction qu'elle soit cytoplasmique, membranaire, ou autre.

L'un des éléments importants au niveau de la sélectivité des Ac mis en oeuvre est que ceux-ci ne soient que peu ou pas présents dans le sérum du patient. Dans la mesure où ces Ag peuvent neutraliser les Ac injectés et provoquer leur capture aspécifique par d'autres cellules cecinécessitera, bien entendu, avant l'application des conjugués comportant les agents de pilotage selon la présente invention une étude sérologique préalable du patient à traiter.

Le second critère mis en évidence dans le cadre de la présente invention est la possibilité pour les Ac d'accéder aux cellules cibles, par exemple aux cellules tumorales. En effet, on a pu constater, dans certains cas, que les anticorps monoclonaux ne pouvaient accéder aux cellules possédant l'Ag correspondant ou bien ne pouvaient y accéder qu'en quantité très faible.

Cette accessibilité peut se faire, soit directement à partir du sang, soit parce que l'agent de pilotage peut franchir certaines barrières vasculaires. D'ailleurs, cette possibilité d'accéder aux cellules cibles peut dépendre également de la présence ou non dans le sérum du patient d'Ag circulants correspondants, comme cela a été dit précédemment, et qui sont susceptibles de limiter l'accès aux cellules cibles.

D'ailleurs, l'accès des Ac à la tumeur, en particulier lorsqu'il s'agit d'anticorps monoclonaux, peut être facilité par l'utilisation de fragments plutôt que de l'immunoglobuline complète.

Le troisième critère de sélection des anticorps monoclonaux est qu'ils puissent être endocytés et aient accès aux lysosomes des cellules cibles. En effet, il est possible de concevoir des liens stables dans le milieu extracellulaire mais hydrolysables par les enzymes lysosomiaux qui permettent de libérer à l'intérieur de la cellule la substance à activité pharmacologique ou à accumulation que l'on désire libérer. Idéalement, il faudrait que l'anticorps soit détaché de son antigène dans les lysosomes sous l'action, soit du pH acide, soit des enzymes, et qu'il soit ensuite digéré. Mais, alternativement, il peut suffire que l'Ac transite par les lysosomes pendant un temps suffisant pour permettre l'hydrolyse du lient unissant l'Ac à la substance pilotée.

Enfin, il est important que ces agents de pilotage qui sont des anticorps n'induisent pas la disparition définitive des antigènes de la membrane cellulaire, ce qui implique d'après endocytose du complexe Ag-Ac il y ait réapparition de l'Ag à la surface des cellules, soit par suite d'une néosynthèse de nouvelles molécules d'Ag, soit par suite du recyclage des Ag endocytés, soit par suite du passage vers la membrane plasmique d'Ag qui sont stockés dans un réservoir intracellulaire.

En effet, il est connu que dans un certain nombre de cas l'interaction d'un Ac, et en particulier lorsque celui-ci est plurivalent, avec son Ag induise la disparition de la surface cellulaire, très probablement par endocytose ; ceci, bien entendu, va limiter le nombre de molécules de conjugués qui pourront reconnaître la cellule cible et être endocytés et,par là même, limiter la concentration intracellulaire pour obtenir l'effet thérapeutique ou diagnostique désiré, ce qui pourra être particulièrement gênant dans le cas de molécules à activité cytotoxique dont on sait qu'elles doivent, pour être actives, être présentes en concentration suffisante dans la cellule et pendant une période suffisamment longue.

Bien entendu, l'utilisation d'Ac ou de fragments d'Ac à titre d'agents de pilotage pourrait, lorsqu'ils sont utilisés in vivo, induire une réaction immunitaire humorale; ainsi il pourrait apparaître des Ac anti-isotype suite à l'administration d'Ac de souris à l'homme, c'est pourquoi on préfèrera, dans la mesure du possible, utiliser des Ac humains qui devraient permettre de lever cet obstacle ; cependant, même dans ce cas, on ne peut exclure, a priori, l'apparition d'Ac anti-allotype et surtout anti-idiotype.

Ce type de réaction immunologique possible devra, bien entendu, être pris en compte dans la réalisation d'un Ac à titre d'agent de pilotage.

Dans le cadre de la présente invention, on utilisera plus particulièrement à titre d'Ac comme agent de pilotage un anticorps monoclonal d'origine murine ou humaine ; de façon encore plus précise, on utilisera un Ac monoclonal dressé contre la membrane de globules graisseux de lait humain et contre des antigènes de la membrane plasmique de cellules de carcinomes humains.

Ces Ac sont plus particulièrement utilisables pour le pilotage de substances à activité anticancéreuse tels des dérivés d'anthracycline ou d'alcaloïdes de vinca.

Dans les exemples qui suivent on décrira plus particulièrement un anticorps dénommé CT-M-01 qui présente l'ensemble des propriétés énumérées précédemment et qui a été testé à titre d'agent de pilotage pour des produits anticancéreux.

La présente invention concerne également des conjugués actifs caractérisés en ce qu'ils comportent un agent de pilotage tel que défini précédemment lié par un lien covalent à une substance à activité pharmacologique ou à accumulation intracellulaire dans des cellules cibles. Ce lien covalent ne sera pratiquement hydrolysé que dans les lysosomes, cette hydrolyse étant le résultat d'une hydrolyse enzymatique acide.

De tels liens covalents sont dejà connus de la technique antérieure et il faut citer, par exemple, les bras hémisuccinyl déjà utilisés pour coupler la vindésine en position $C_4$ et le bras tétrapeptidique déjà utilisé pour coupler la daunorubicine, la doxorubicine et la primaquine. Mais, d'autres liens peuvent être envisagés sans sortir du cadre de la présente invention.

Lorsque l'Ac monoclonal est plus particulièrement destiné à piloter un médicament anticancéreux, ce médicament anticancéreux sera de préférence, mais de manière non exclusive, choisi dans la famille des dérivés d'anthracycline ou d'alcaloïdes de vinca, par exemple parmi la vinblastine, la vindésine, la daunorubicine ou la doxorubicine.

Pour la réalisation de ces conjugués, il convient de vérifier que,par hydrolyse dans les lysosomes, le médicament ou le produit de diagnostic sera libéré sous une forme active, c'est-à-dire que le lien de covalence n'est pas susceptible de masquer l'activité du produit, et il faudra vérifier, d'autre part, que le lien de covalence ne modifie pas les sites de reconnaissance spécifiques de l'Ac utilisé.

Il convient de vérifier également que les dimensions ou les propriétés des molécules actives liées aux Ac sont telles qu'elles leur permettent de franchir les différentes barrières leur permettant d'accéder au lysosomes.

Les études effectuées qui seront rapportées ci-après dans les exemples ont mis, en outre, en évidence l'importance de la concentration intracellulaire en médicament suffisante pour obtenir l'effet cytotoxique. En effet, pour obtenir une concentration suffisante, il est possible de jouer sur deux paramètres, soit la drogue est très active, c'est-à-dire requiert des concentrations intracellulaires relativement faibles, et à ce moment il devient possible d'utiliser un Ac reconnaissant l'Ag présent à la surface cellulaire en quantité relativement réduite, soit il est nécessaire de réaliser une concentration intracellulaire élevée en médicament, comme par exemple dans le cas de la daunorubicine, et dans ce cas il faut utiliser un Ac reconnaissant un Ag présent en plus grande quantité sur la membrane plasmique des cellules et/ou recyclant continuellement et permettant ainsi de faire endocyter plus de molécules d'Ac par Ag exposé.

Cet aspect du problème doit, dans une certaine mesure, faire reconsidérer le problème de la sélectivité de l'Ac. En effet, si l'Ag reconnu par l'Ac monoclonal est très spécifique de la cellule tumorale, il sera fort probablement présent en très petit nombre et dans ces conditions ne permettra pas d'obtenir des concentrations intracellulaires élevées en médicament.

On conçoit à la lecture de la description précédente que les agents de pilotage selon l'invention peuvent être utilisés à titre d'agents de diagnostic dans le domaine de l'imagerie médicale et de la radiothérapie en couplant un radio-isotope par exemple de manière covalente à l'Ac, ledit lien étant lui-même hydrolysable dans les mêmes conditions que celles décrites précédemment, bien que le problème de l'hydrolyse du lien unissant le radio-isotope et l'Ac soit moins important que dans le cas d'un composé à activité pharmacologique.

Les exemples ci-après sont destinés à illustrer d'autres caractéristiques et avantages de la présente invention sans pour autant la limiter en aucune façon.

## EXEMPLE 1

### Préparation de l'anticorps monoclonal CT-M-01

Le protocole expérimental utilisé pour la préparation des Ac monoclonaux dressés contre la membrane de globules graisseux de lait humain est comparable à celui qui a été décrit par Taylor-Papadimitriou et al. (1981).

La fusion d'une lignée cellulaire de myélome murin ne sécrétant pas d'immunoglobuline,et dénommée SP 207, avec les lymphocytes spléniques de souris immunisées avec des membranes de globules

5

graisseux de lait humain (la fusion étant effectuée 3 et 5 mois après la dernière injection subcutanée) conduit à la production d'un grand nombre de cultures d'hybridomes sécrétant des immunoglobulines.

La sélection des hybridomes a été effectuée en testant, tout d'abord, leur réaction d'anticorps en utilisant les antigènes correspondants immobilisés, puis la sous-classe d'Ac a été déterminée, on a éliminé les hybridomes sécrétant des IgM, puis on a étudié la réactivité avec la membrane de cellules MCF-7 en culture (cellules d'un carcinome humain de la glande mammaire) et on a, enfin, sélectionné les cultures d'hybridomes qui maintenaient une sécrétion d'IgG réactives à la fois avec la membrane de globules graisseux de lait humain et de cellules MCF-7, même après congélation à - 80°C.

Ces cultures d'hybridomes ont été administrées en injection intrapéritonéale à des souris et les Ac ont été purifiés à partir des ascites qui se sont développées.

On a, ainsi, retenu 6 anticorps monoclonaux de sous-classes différentes pour une étude plus complète qui ont été dénommés : CT-M-01 (sous-classe $IgG_2$ B), CT-M-02 ($IgG_{2b}$), CT-M-03 ($IgG_1$), CT-M-04 ($IgG_3$), CT-M-05 ($IgG_3$), et CT-M-06 ($IgG_3$).

## EXEMPLE 2

### Etude des propriétés des anticorps monoclonaux

Les différents anticorps monoclonaux préparés à l'exemple 1 ont, tout d'abord, été étudiés quant à la distribution des épitopes sur les tissus humains normaux (tableau IA ci-annexé) et néoplasiques (tableau IB ci-annexé) fixés à la formaldéhyde et enrobés à la paraffine par des techniques d'immunohistologie indirecte à l'aide d'un second anticorps anti-IgG de souris, marqué à la peroxydase.

D'autres essais complémentaires effectués ont montré que dans tous les cas l'Ac CT-M-01 était le seul des Ac étudiés à reconnaître les Ag de façon uniforme à la surface de toutes les cellules néoplasiques d'origine mammaire, tumeurs primaires et embolies tumorales des vaisseaux veineux et lymphatiques, de même que dans les métastases lymphatiques axillaires dans tous les cas testés.

Les 5 autres Ac marquent de façon aléatoire et hétérogène les cellules néoplasiques avec des profils particuliers pour chacun d'eux.

## TABLEAU 1.A.

**REACTION CROISEE D'ANTICORPS MONOCLONAUX DIRIGES CONTRE LA MEMBRANE DES GLOBULES GRAISSEUX DU LAIT HUMAIN AVEC DES TISSUS HUMAINS NORMAUX**

|  | CT-M-01 | 02 | 03 | 04 | 05 | 06 |
|---|---|---|---|---|---|---|
| ESTOMAC : EPITHELIUM DE SURFACE | a | a | a | a | a | a |
| GLANDES GASTRIQUES | a | d | Neg. | Neg. | d | d |
| INTESTIN GRELE : EPITHELIUM DE SURFACE | Neg. | a | a | d | c | d |
| CRYPTES DE LIEBERKUHN | a | d | a | d | d | d |
| GLANDES DE BRUNNER | a | d | d | d | d | d |
| PANCREAS EXOCRINE : ACINI | Neg. | Neg. | c | c | d | a |
| CANAUX | b | a | a | b | b | a |
| FOIE : CANALICULES BILIAIRES | b | b | a | b | b | b |
| COLON : EPITHELIUM DE SURFACE ET CRYPTES | Neg. | d | a | Neg. | Neg. | d |
| APPENDICE : EPITHELIUM DE SURFACE ET CRYPTES | Neg. | b | c | b | b | a |
| GLANDES SALIVAIRES : ACINI | Neg. | d | b | b | c | b |
| CANAUX | d | a | b | b | b | b |
| REIN : TUBES COLLECTEURS | a | Neg. | Neg. | Neg. | Neg. | Neg. |
| TRACHEE : EPITHELIUM RESPIRATOIRE | Neg. | d | Neg. | d | d | d |
| GLANDES DE LA MUQUEUSE | b | b | b | b | b | b |
| TROMPE DE FALLOPE : EPITHELIUM DE SURFACE | a | d | b | c | b | d |
| ENDOMETRE : SURFACE ET GLANDES | a | Neg. | c | Neg. | Neg. | d |
| COL UTERIN : EXOCOL | Neg. | a | a | a | a | a |
| ENDOCOL | a | a | a | a | a | a |
| PEAU : GLANDES SUDORIPARES | a | d | d | c | Neg. | b |
| GLANDES SEBACEES | a | Neg. | Neg. | Neg. | Neg. | Neg. |

EP 0 208 615 B1

## TABLEAU 1.A. (SUITE)

| | | | | | | |
|---|---|---|---|---|---|---|
| GLANDE MAMMAIRE AU REPOS : CANALICULES TERMINAUX | a | b | b | c | b | b |
| CANALICULES INTERLOBULAIRES | a | b | b | d | b | b |
| CANALICULES GALACTOPHORES | a | Neg. | b | c | Neg. | d |
| MOELLE OSSEUSE : | $a^1$ | Neg. | Neg. | Neg. | Neg. | Neg. |
| GLANDES THYROIDES : CELLULES FOLLICULAIRES | d | Neg. | Neg. | Neg. | Neg. | Neg. |
| COLLOIDES | d | Neg. | Neg. | d | Neg. | d |
| CANAL DEFERENT : EPITHELIUM | a | Neg. | Neg. | Neg. | Neg. | Neg. |
| GLANDE PROSTATIQUE | d | Neg. | d | d | d | d |
| CERVEAU, CERVELET, TISSU CONJONCTIF, ENDOTHELIUM, OVAIRE, EPIDERME, VESICULE BILIAIRE, HEPATOCYTES, MUSCLES, ILOTS DE LANGHERANS, TISSU LYMPHATIQUE, GLOMERULES RENAUX, MYOEPITHELIUM, GLANDES SURRENALES, RETINE, MOELLE EPINIERE | Neg. | Neg. | Neg. | Neg. | Neg. | Neg. |

1 : MONOCYTES

MARQUAGE : a : uniforme
           b : légèrement hétérogène (la plupart des cellules sont marquées)
           c : moyen
           d : fortement hétérogène (la plupart des cellules ne sont pas marquées)
    Neg.: négatif (pas de marquage)

EP 0 208 615 B1

TABLEAU 1.B.

| REACTION IMMUNOHISTOCHIMIQUE DES ANTICORPS MONOCLONAUX DIRIGES CONTRE LA MEMBRANE DES GLOBULES GRAISSEUX DU LAIT HUMAIN AVEC DIFFERENTS TISSUS NEOPLASIQUES HUMAINS | | | | | | |
|---|---|---|---|---|---|---|
| | CT-M-01 | 02 | 03 | 04 | 05 | 06 |
| CARCINOME CANALAIRE DU SEIN | a | d | c | d | b | b |
| CARCINOME LOBULAIRE DU SEIN | a | d | c | d | c | c |
| CARCINOME RENAL | a | Neg. | Neg. | Neg. | Neg. | Neg. |
| CARCINOME DE LA VESSIE | c | d | b | d | c | d |
| HEPATOCARCINOME | Neg. | Neg. | Neg. | Neg. | Neg. | Neg. |
| CARCINOME EPIDERMOIDE DU COL UTERIN | b | d | c | d | c | d |
| CARCINOME EPIDERMOIDE DE L'OESOPHAGE | Neg. | Neg. | Neg. | Neg. | Neg. | Neg. |
| CARCINOME EPIDERMOIDE DE LA PEAU | Neg. | Neg. | Neg. | Neg. | Neg. | Neg. |
| CARCINOME BASAL DE LA PEAU | Neg. | Neg. | Neg. | Neg. | Neg. | Neg. |
| ADENOCARCINOME DE L'ESTOMAC | c | b | a | d | a | d |
| ADENOCARCINOME DU COLON | c | d | a | b | b | b |
| ADENOCARCINOME DE L'ENDOMETRE | a | d | c | c | c | b |
| CYSTADENOCARCINOME ENDOMETROIDE DE L'OVAIRE | a | d | b | c | c | b |
| MELANOME DE LA PEAU | Neg. | Neg. | Neg. | Neg. | Neg. | Neg. |
| LYMPHOME B | Neg. | Neg. | Neg. | Neg. | Neg. | Neg. |
| LIPOSARCOME | Neg. | Neg. | Neg. | Neg. | Neg. | Neg. |

Même échelle de marquage que pour le tableau 1.A.

EXEMPLE 3

Interaction des anticorps monoclonaux avec les cellules MCF-7

L'étude de la fixation des Ac monoclonaux par les cellules MCF-7 est effectué en utilisant un marquage à l'hydrogène tritié des anticorps monoclonaux.

Pour ce faire, une culture confluente de cellules MCF-7 ($2.10^6$ cellules dans des flacons de 25 $cm^2$) est incubée à 4°C pendant 3 heures en présence de différentes concentrations d'anticorps monoclonaux marqués. A la fin de l'incubation la quantité de marqueur radioactif associé aux cellules est déterminée. La constante d'affinité et le nombre de sites antigéniques par cellule caractérisant les différents Ac monoclonaux est calculée par analyse de Scatchard des paramètres de fixation. Le nombre des sites antigéniques par cellule est estimé en attribuant 1 mg de protéines cellulaires à $3.10^6$ cellules MCF-7 et en tenant compte d'une stoechiométrie 1:1 pour la liaison entre les AC et leurs épitopes.

Les résultats figurent au tableau II ci-annexé.

Les études suivantes ont porté plus particulièrement sur l'étude de l'anticorps CT-M-01.

L'étude de la distribution de l'Ac marqué dans des cultures de cellules MCF-7 confluentes a permis de mettre en évidence que cet Ac était endocyté, qu'il se détachait à pH acide et qu'il rejoignait les lysosomes où il est digéré (tableau III ci-annexé).

Ces résultats ont d'ailleurs été confirmés par l'effet d'un certain nombre de substances connues pour interférer avec l'endocytose et la fonction digestive des lysosomes.

TABLEAU II

| ANALYSE SELON LA METHODE DE SCATCHARD DES PARAMETRES DE FIXATION D'ANTICORPS MONOCLONAUX DIRIGES CONTRE LA MEMBRANE DES GLOBULES GRAISSEUX DU LAIT HUMAIN A DES CELLULES D'UN CARCINOME MAMMAIRE HUMAIN (LIGNEE MCF-7) | | |
|---|---|---|
| Anticorps monoclonal | Kd (M) | nombre de sites antigéniques par cellule |
| CT-M-01 | $1.39.10^{-8}$ | $1.2.10^6$ |
| CT-M-02 | $6.26.10^{-7}$ | $17.0.10^6$ |
| CT-M-03 | $2.95.10^{-8}$ | $1.6.10^6$ |
| CT-M-04 | $3.02.10^{-8}$ | $4.6.10^6$ |
| CT-M-05 | $1.50.10^{-7}$ | $7.8.10^6$ |
| | $5.20.10^{-8}$ | $4.4.10^6$ |
| CT-M-06 | $3.16.10^{-9}$ | $8.1.10^4$ |
| | $6.20.10^{-7}$ | $5.6.10^6$ |

Des cellules confluentes de la lignée MCF-7 ($2.10^6$ cellules par flacon de 25 cm$^2$) ont été incubées à 4°C pendant 3 heures en présence de différentes concentrations des anticorps monoclonaux marqués au $^3$H. A la fin de l'incubation, la quantité de matériel radioactif associé aux cellules a été déterminée. Les constantes d'affinité et le nombre de sites antigéniques par cellule ont été déterminés pour chaque anticorps selon la méthode de Scatchard (1949). Le nombre de sites antigéniques a été estimé en attribuant 1 mg de protéine cellulaire à $3.10^6$ cellules MCF-7 et en considérant une stéchiométrie 1:1 pour la fixation des anticorps à leurs épitopes.

TABLEAU III

| PARAMETRES CINETIQUES DE LA CAPTURE DE SIX ANTICORPS MONOCLONAUX DIRIGES CONTRE LA MEMBRANE DES GLOBULES GRAISSEUX DU LAIT HUMAIN PAR DES CELLULES DE TUMEURS DE LA GLANDE MAMMAIRE (LIGNEE MCF-7) | | | | |
|---|---|---|---|---|
| anticorps monoclonal | K capture | nombre de molécules d'IgG/cell/14 h. | | |
| | | accumulation | digestion | capture |
| CT-M-01 | $4.9.10^{-7}$ | $7.0.10^6$ | $2.4.10^6$ | $9.4.10^6$ |
| CT-M-02 | $16.7.10^{-7}$ | $3.0.10^6$ | $2.5.10^6$ | $5.5.10^6$ |
| CT-M-03 | $8.6.10^{-7}$ | $1.7.10^6$ | - | $1.7.10^6$ |
| CT-M-04 | $3.4.10^{-7}$ | $4.5.10^6$ | $3.4.10^6$ | $7.9.10^6$ |
| CT-M-05 | $5.6.10^{-7}$ | $4.4.10^6$ | $3.7.10^6$ | $8.1.10^6$ |
| CT-M-06 | - | $5.0.10^6$ | $1.0.10^6$ | $6.0.10^6$ |

Des cellules MCF-7 confluentes ($2.10^6$ par flacon de 25 cm$^2$) ont été incubées à 37°C pendant 24 heures en présence de différentes concentrations de chacun des anticorps monoclonaux marqués. A la fin de l'incubation, on a déterminé le nombre de molécules radioactives associées aux cellules (accumulation) digérées (retrouvées dans le milieu de culture sous forme de fragments marqués solubles dans l'acide trichloroacétique à 15 %) ou captées (somme de l'accumulation et de la digestion). Le K capture correspond à la demi saturation de la courbe de capture Le nombre de molécules d'anticorps interréagissant avec chaque cellule MCF-7 a été calculé en attribuant 1 mg de protéine cellulaire à $3.10^6$ cellules MCF-7.

EXEMPLE 4

C'est cet Ac monoclonal CT-M-01 qui est utilisé pour servir d'agent de pilotage dans les conjugués avec des substances à activité cytotoxique.

Parmi les dérivés à activité cytotoxique, on a choisi des dérivés de type anthracyclique, c'est-à-dire la daunorubicine (DNR) et la doxorubicine (DOX) et des dérivés de vinca, en particulier la vinblastine (VBL) et la vindésine (VDS).

Tous ces produits sont déjà connus pour présenter une activité antitumorale.

Les conjugués selon la présente invention sont préparés en utilisant la méthode dite à l'hémisuccinate, dans le cas de la vindésine, par les procédés décrits dans le brevet européen n° 0 124 502 et l'article de K.S.P. Bhushana Rao et al., 1985, et par l'intermédiaire d'un bras tétrapeptidique L-Leu-L-Ala-L-Leu-L-Ala-succinyl, dans le cas de la daunorubicine, comme cela est décrit dans les brevets américain n° 4 376 765, européen n° 0 037 388 et dans les articles de A. Trouet et al., 1982, et B. Lesur et al., 1984.

Dans un premier temps, on a vérifié que le conjugué selon l'invention conservait en grande partie son affinité d'anticorps pour les antigènes présents à la surface des cellules MCF-7.

Ensuite, on a vérifié que les conjugués selon l'invention libéraient bien le principe actif après incubation en présence d'enzymes lysosomaux.

Ainsi, pour le conjugué DNR/CT-M-01, 32,5 % de la DNR est libéré sous forme intacte au bout de 6 heures d'incubation, ce taux de libération de la DNR atteint 60 % au bout de 24 heures d'incubation.

Pour le conjugué VDS/CT-M-01, 33 % du principe actif est libéré au bout de 6 heures d'incubation, aucune hydrolyse supplémentaire d'intervient par la suite.

EXEMPLE 5

Activité des conjugués selon l'invention

Une culture non confluente de cellules E dérivées de la lignée MCF-7 ou de cellules d'hépatoblastomes humains HEPG2 est incubée pendant 6 jours à 37°C en présence de différentes concentrations de DNR/CT-M-01 ou de VDS/CT-M-01 (rapport molaire 1:2 et 1:4,5 respectivement), ainsi qu'avec l'Ac CT-M-01 seul à des concentrations analogues et également avec les drogues libres.

A la fin des 6 jours d'incubation, les cellules sont lavées et testées pour leur contenu en protéines.

Les résultats sont présentés dans le tableau IV sous forme du pourcentage de protéines associées aux cellules pour les cellules traitées par rapport aux cellules non traitées en fonction de la concentration de drogues conjuguées ou libres dans le milieu extracellulaire.

L'Ac non conjugué CT-M-01 est incapable de produire un effet cytotoxique dans les cellules MCF-7. Le conjugué VDS/CT-M-01 présente un effet cytotoxique marqué sur le carcinome mammaire, alors qu'aucune activité cytotoxique n'est observée pour les cellules de l'hépatocarcinome (lignée cellulaire HEPG2) dans les mêmes conditions expérimentales.

La moitié des cellules de la lignée E sont tuées par une concentration extracellulaire de 75 ng/ml de VDS conjugué à CT-M-01. La concentration d'Ac à cette dose correspond à environ 1,102 $\mu$g/ml dans le milieu extracellulaire.

## TABLEAU IV

EFFET CYTOTOXIQUE DE LA DAUNORUBICINE (DNR) ET DE LA VINDESINE (VDS) LIBRES OU CONJUGUEES A DES ANTICORPS MONOCLONAUX SUR DES CELLULES EN CULTURE

| DROGUE | DUREE DE L'EXPOSITION | LD50 (ng/ml (1)) cellules HepG2 | cellules MCF-7 (lignée parentale) | cellules MCF-7 (sousclone E) |
|---|---|---|---|---|
| DNR | 24 h. | - (2) | 20 | - |
| | 6 j. | 44 | - | 68 |
| DNR-CT-M-01 IgG | 6 j. | >360 | - | > 360 |
| VDS | 1 h. | - | - | 100 |
| | 3 h. | - | - | 62 |
| | 6 h. | - | - | 38 |
| | 1 j. | - | 12.7 | 19 |
| | 6 j. | 3.4 | - | 1.1 |
| VDS-CT-M-01 IgG | 3 h. | - | - | >1000 |
| | 6 h. | - | - | 800 |
| | 1 j. | - | - | 98 |
| | 3 j. | - | - | 45 |
| | 6 j. | >872 | - | 52 |
| VDS-IgG(3) | 1 j. | - | - | >1000 |
| | 3 j. | - | - | >1000 |
| | 6 j. | - | - | 1000 |

(1) : concentration extracellulaire de drogue libre ou conjuguée requise pour diminuer la quantité de protéines cellulaires après 6 jours de culture à 50 % de celle atteinte par des cellules cultivées en absence de drogue.

(2) - : valeur non déterminée.

(3) : IgG à spécificité non déterminée.

L'absence de résultats déterminants observée avec le conjugué DNR dans un modèle in vitro peut trouver son origine dans le fait que la DNR ne peut pas atteindre son site d'activité intracellulaire en quantité suffisante pour que l'on observe une activité cytotoxique.

Pour ce qui concerne le conjugué VDS/CT-M-01, on a démontré qu'il était capable de tuer sélectivement les cellules de carcinome mammaire sans affecter les cellules qui ne portent pas les antigènes

EP 0 208 615 B1

correspondants à l'anticorps.

Des essais complémentaires ont permis de mettre en évidence que cette activité ne pouvait pas être attribuée au produit cytotoxique libre.

**Revendications**

**Revendications pour les Etats contractants suivants : BE, CH, DE, FR, GB, IT, LI, LU, NL, SE**

1. A titre d'agent de pilotage de substances à activité pharmacologique ou de substances utiles à des fins diagnostiques, dans des cellules cibles de carcinomes humains, un anticorps (Ac) ou un fragment d'anticorps caractérisé en ce que :
   a) il s'agit d'un anticorps monoclonal dressé contre la membrane de globules graisseux de lait humain et contre la membrane plasmique de cellules cibles de carcinomes humains,
   b) les antigènes (Ag) correspondants ne sont pratiquement accessibles qu'à la surface des cellules cibles,
   c) l'Ac ou le fragment d'Ac peut accéder aux cellules cibles,
   d) l'Ac ou le fragment d'Ac est endocyté et à accès aux lysosomes des cellules cibles,
   e) l'Ac ou le fragment d'Ac n'induit pas la disparition définitive des Ag correspondants de la surface des cellules cibles.

2. Agent de pilotage selon la revendication 1, caractérisé en ce qu'il est constitué par une molécule entière d'immunoglobine ou par un fragment d'anticorps F(ab), F(ab') ou F(ab')$_2$.

3. Agent de pilotage selon l'une des revendications 1 et 2, caractérisé en ce que le sérum du patient à traiter comporte peu ou pas d'Ag correspondants.

4. Agent de pilotage selon l'une des revendications 1 à 3, caractérisé en ce qui s'agit d'un Ac monoclonal d'origine murine ou humaine.

5. Agent de pilotage selon la revendication 4, caractérisé en ce qu'il s'agit de l'anticorps CT-M-01.

6. Conjugué actif, caractérisé en ce qu'il comporte un agent de pilotage selon l'une des revendications 1 à 5, lié par un lien covalent à une substance à activité pharmacologique ou une substance utile à des fins diagnostiques dans des cellules tumorales.

7. Conjugué actif selon la revendication 6, caractérisé en ce que le lien covalent n'est en pratique hydrolysé que dans les lysosomes.

8. Conjugué actif selon la revendication 7, caractérisé en ce que l'hydrolyse est une hydrolyse enzymatique acide.

9. Conjugué actif selon l'une des revendications 6 à 8, caractérisé en ce que le composant est un médicament anticancéreux.

10. Conjugué actif selon la revendication 9, caractérisé en ce que le médicament anticancéreux est choisi dans la famille des dérivés d'anthracyclines ou d'alcaloïdes vinca.

11. Conjugué actif selon la revendication 10, caractérisé en ce que le composant est choisi parmi la vindésine, la daunorubicine et la doxorubicine.

12. Conjugué actif selon l'une des revendications 6 à 11, caractérisé en ce que le lien covalent est un bras hémisuccinyl ou tétrapeptidique.

13. Conjugué actif selon l'une des revendications 6 à 12, caractérisé en ce que le composant actif est choisi parmi la vindésine, la daunorubicine, la doxorubicine et l'agent de pilotage est l'anticorps CT-M-01.

**Revendications pour l'Etat contractant suivant : AT**

16

EP 0 208 615 B1

1. Procédé de préparation d'un agent de pilotage de substances à activité pharmacologique ou de substances utiles à des fins diagnostiques, dans des cellules cibles de carcinomes humains, caractérisé en ce qu'on utilise un anticorps (Ac) ou un fragment d'anticorps sélectionné comme suit :

a) il s'agit d'un anticorps monoclonal dressé contre la membrane de globules graisseux de lait humain et contre la membrane plasmique de cellules cibles de carcinomes humains,

b) les antigènes (Ag) correspondants ne sont pratiquement accessibles qu'à la surface des cellules cibles,

c) l'Ac ou le fragment d'Ac peut accéder aux cellules cibles,

d) l'Ac ou le fragment d'Ac est endocyté et à accès aux lysosomes des cellules cibles,

e) l'Ac ou le fragment d'Ac n'induit pas la disparition définitive des Ag correspondants de la surface des cellules cibles.

2. Procédé selon la revendication 1, caractérisé en ce qu'il est constitué par une molécule entière d'immunoglobine ou par un fragment d'anticorps F(ab), F(ab') ou F(ab')$_2$.

3. Procédé selon les revendications 1 et 2, caractérisé en ce que le sérum du patient à traiter comporte peu ou pas d'Ag correspondants.

4. Procédé selon l'une des revendications 1 à 3, caractérisé en ce qu'il s'agit d'un Ac monoclonal d'origine murine ou humaine.

5. Procédé selon la revendication 4, caractérisé en ce qu'il s'agit de l'anticorps CT-M-01.

6. Procédé de préparation d'un conjugué actif, caractérisé en ce qu'on couple un agent de pilotage selon l'une des revendications 1 à 5, par un lien covalent à une substance à activité pharmacologique ou une substance utile à des fins diagnostiques dans des cellules tumorales.

7. Procédé selon la revendication 6, caractérisé en ce que le lien covalent n'est en pratique hydrolysé que dans les lysosomes.

8. Procédé selon la revendication 7, caractérisé en ce que l'hydrolyse est une hydrolyse enzymatique acide.

9. Procédé selon l'une des revendications 6 à 8, caractérisé en ce que le composant est un médicament anticancéreux.

10. Procédé selon la revendication 9, caractérisé en ce que le médicament anticancéreux est choisi dans la famille des dérivés d'anthracyclines ou d'alcaloïdes vinca.

11. Procédé selon la revendication 10, caractérisé en ce que le composant est choisi parmi la vindésine, la daunorubicine et la doxorubicine.

12. Procédé selon l'une des revendications 6 à 11, caractérisé en ce que le lien covalent est un bras hémisuccinyl ou tétrapeptidique.

13. Procédé selon l'une des revendications 6 à 12, caractérisé en ce que le composant actif est choisi parmi la vindésine, la daunorubicine, la doxorubicine et l'agent de pilotage est l'anticorps CT-M-01.

**Claims**

**Claims for the following Contracting States : BE, CH, DE, FR, GB, IT LI, LU, NL, SE**

1. By way of a targeting agent for substances which show pharmacological activity or substances useful for diagnostic in human carcinoma target cells, an antibody (Ab) or an antibody fragment, defined as follows :

a) it is a monoclonal antibody raised against the membrane of fat globules of human milk and against the plasma membrane of human carcinoma target cells,

b) the corresponding antigens (Ag's) are, in practice, accessible only at the surface of the target cells,

17

c) the Ab or Ab fragment can gain access to the target cells,
d) the Ab or Ab fragment is endocytosed and has access to the lysosomes of the target cells,
e) the Ab or Ab fragment does not induce the permanent disappearance of the corresponding Ag's of the surface of the target cells.

2. The targeting agent as claimed in claim 1, which consists of a whole immunoglobulin in molecule or of a Fa, Fab' or F(ab')$_2$ antibody fragment.

3. The targeting agent as claimed in one of claims 1 and 2, in which the serum of the patient to be treated contains few or no corresponding Ag's.

4. The targeting agent as claimed in one of claims 1 to 3, which is a monoclonal Ab or murine or human origin.

5. The targeting agent as claimed in one of claim 1 to 4, which is the antibody CT-M-01.

6. An active conjugate, which contains a targeting agent as claimed in one of claims 1 to 5, linked through a covalent bond to a substance which shows pharmacological activity or intracellular accumulation in tumor cells.

7. The active conjugate as claimed in claim 6, in which the covalent bond is, only hydrolyzed in the lysosomes.

8. The active conjugate as claimed in claim 7, in which the hydrolysis is an acid enzymatic hydrolysis.

9. The active conjugate as claimed in one of claims 6 to 8, in which the component is an anticancer drug.

10. The active conjugate as claimed in claim 9, in which the anticancer drug is chosen from the family of anthracycline derivatives or Vinca alkaloid derivatives.

11. The active conjugate as claimed in claim 10, in which the component is chosen from vindesine, daunorubicin and doxorubicin.

12. The active conjugate as claimed in one of claims 6 to 11, in which the covalent bond is a hemisuccinyl or tetrapeptide branch.

13. The active conjugate as claimed in one of claims 6 to 12, in which the active component is chosen from vindesine, daunorubicin and doxorubicin and the targeting agent is the antibody CT-M-01.

**Claims for the following Contracting State : AT**

1. Process for the preparation of a targeting agent for substances which show pharmacological activity or substances useful for diagnostic in human carcinoma target cells, characterized in that an antibody (Ab) or an antibody fragment is used defined as follows :
   a) it is a monoclonal antibody raised against the membrane of fat globules of human milk and against the plasma membrane of human carcinoma target cells,
   b) the corresponding antigens (Ag's) are, in practice, accessible only at the surface of the target cells,
   c) the Ab or Ab fragment can gain access to the target cells,
   d) the Ab or Ar fragment is endocytosed and has access to the lysosomes of the target cells,
   e) the Ab or Ab fragment does not induce the permanent disappearance of the corresponding Ag's of the surface of the target cells.

2. The process as claimed in claim 1, which consists of a whole immunoglobulin molecule or of a Fab, Fab' or F(ab')$_2$ antibody fragment.

3. The process as claimed in one of claims 1 and 2, in which the serum of the patient to be treated contains few or no corresponding Ag's.

EP 0 208 615 B1

**4.** The process as claimed in one of claims 1 to 3, which is a monoclonal Ab of murine or human origin.

**5.** The process as claimed in one of claims 1 to 4, which is the antibody CT-M-01.

**6.** Process for the preparation of an active conjugate, characterized in that a targeting agent as claimed in one of claims 1 to 5, is linked through a covalent bond to a substance which shows pharmacological activity or intracellular accumulation in tumor cells.

**7.** The process as claimed in claim 6, in which the covalent bond is, only hydrolyzed in the lysosomes.

**8.** The process as claimed in claim 7, in which the hydrolysis is an acid enzymatic hydrolysis.

**9.** The process as claimed in one of claims 6 to 8, in which the component is an anticancer drug.

**10.** The process as claimed in claim 9, in which the anticancer drug is chosen from the family of anthracycline derivatives or Vinca alkaloid derivatives.

**11.** The process as claimed in claim 10, in which the component is chosen from vindesine, daunorubicin and doxorubicin.

**12.** The process as claimed in one of claims 6 to 11, in which the covalent bond is a hemisuccinyl or tetrapeptide branch.

**13.** The process as claimed in one of claims 6 to 12, in which the active component is chosen from vindesine, daunorubicin and doxorubicin and the targeting agent is the antibody CT-M-01.

**Patentansprüche**
**Patentansprüche für folgende Vertragsstaaten : BE, CH, DE, FR, GB, IT, LI, LU, NL, SE**

**1.** Mittel zur Steuerung (Lenkung) von Substanzen mit einer pharmakologischen Aktivität oder von Substanzen, die für diagnostische Zwecke verwendbar sind, in Human-Carcinom-Targetzellen in Form eines Antikörpers (Ac) oder eines Antikörper-Fragments, dadurch gekennzeichnet, daß
a) es sich handelt um einen monoklonalen Antikörper, der gerichtet ist gegen die Fettkügelchen-Membran von Human-Milch und gegen die Plasma-Membran von Human-Carcinom-Targetzellen,
b) die entsprechenden Antigene (Ag) praktisch nur an der Oberfläche der Targetzellen zugänglich sind,
c) der Antikörper oder das Antikörper-Fragment zu den Targetzellen gelangen kann,
d) der Antikörper oder das Antikörper-Fragment endocytiert wird und Zugang zu den Lysosomen der Targetzellen hat und
e) der Antikörper oder das Antikörper-Fragment das endgültige Verschwinden der entsprechenden Antigene von der Oberfläche der Targetzellen nicht induziert.

**2.** Steuerungsmittel nach Anspruch 1, dadurch gekennzeichnet, daß es besteht aus einem vollständigen Immunoglobulin-Molekül oder einem Antikörper-Fragment F(ab), F(ab') oder F(ab')$_2$.

**3.** Steuerungsmittel nach einem der Ansprüche 1 und 2, dadurch gekennzeichnet, daß das Serum des zu behandelnden Patienten wenige oder keine entsprechenden Antigene enthält.

**4.** Steuerungsmittel nach einem der Ansprüche 1 bis 3, dadurch gekennzeichnet, daß es sich dabei um einen monoklonalen Antikörper handelt, der von der Maus oder vom Menschen stammt.

**5.** Steuerungsmittel nach Anspruch 4, dadurch gekennzeichnet, daß es sich dabei handelt um den Antikörper CT-M-01.

**6.** Aktives Konjugat, dadurch gekennzeichnet, daß es ein Steuerungsmittel nach einem der Ansprüche 1 bis 5 enthält, das über ein covalentes Bindeglied an eine Substanz mit pharmakologischer Aktivität oder an eine Substanz, die für diagnostische Zwecke in Tumorzellen verwendbar ist, gebunden ist.

19

**7.** Aktives Konjugat nach Anspruch 6, dadurch gekennzeichnet, daß das covalente Bindeglied in der Praxis nur in den Lysosomen hydrolysiert wird.

**8.** Aktives Konjugat nach Anspruch 7, dadurch gekennzeichnet, daß die Hydrolyse eine enzymatische Säurehydrolyse ist.

**9.** Aktives Konjugat nach einem der Ansprüche 6 bis 8, dadurch gekennzeichnet, daß die Substanz (Komponente) ein Antikrebs-Arzneimittel ist.

**10.** Aktives Konjugat nach Anspruch 9, dadurch gekennzeichnet, daß das Antikrebs-Arzneimittel ausgewählt wird aus der Familie der Derivate von Anthracyclinen oder Vinca-Alkaloiden.

**11.** Aktives Konjugat nach Anspruch 10, dadurch gekennzeichnet, daß die Substanz (Komponente) ausgewählt wird aus Vindesin, Daunorubicin und Doxorubicin.

**12.** Aktives Konjugat nach einem der Ansprüche 6 bis 11, dadurch gekennzeichnet, daß das kovalente Bindeglied ein Hemisuccinyl-Zweig oder ein Tetrapeptid-Zweig ist.

**13.** Aktives Konjugat nach einem der Ansprüche 6 bis 12, dadurch gekennzeichnet, daß die aktive Substanz (Komponente) ausgewählt wird aus Vindesin, Daunorubicin und Doxorubicin und daß das Steuerungsmittel der Antikörper CT-M-01 ist.

**Patentansprüche für folgenden Vertragsstaat : AT**

**1.** Verfahren zur Herstellung eines Mittels zur Steuerung (Lenkung) von Substanzen mit pharmakologischer Aktivität oder von Substanzen, die für diagnostische Zwecke verwendbar sind, in Human-Carcinom-Targetzellen, dadurch gekennzeichnet, daß man einen Antikörper (Ac) oder ein Antikörper-Fragment verwendet, der (das) wie folgt ausgewählt wird:
a) es handelt sich dabei um einen monoklonalen Antikörper, der gegen die Fettkügelchen-Membran von Human-Milch und gegen die Plasma-Membran von Human-Carcinom-Targetzellen gerichtet ist,
b) die entsprechenden Antigene (Ag) sind praktisch nur an der Oberfläche der Targetzellen zugänglich,
c) der Antikörper oder das Antikörper-Fragment kann zu den Targetzellen gelangen,
d) der Antikörper oder das Antikörper-Fragment wird endocytiert ist und hat Zugang zu den Lysosomen der Targetzellen und
e) der Antikörper oder das Antikörper-Fragment induziert das endgültige Verschwinden der entsprechenden Antigene von der Oberfläche der Targetzellen nicht.

**2.** Verfahren nach Anspruch 1, dadurch gekennzeichnet, daß es besteht aus einem vollständigen Immunoglobulin-Molekül oder aus einem Antikörper-Fragment F)ab), F(ab') oder F(ab')$_2$.

**3.** Verfahren nach den Ansprüchen 1 und 2, dadurch gekennzeichnet, daß das Serum des zu behandelnden Patienten wenige oder keine entsprechenden Antigene enthält.

**4.** Verfahren nach einem der Ansprüche 1 bis 3, dadurch gekennzeichnet, daß es sich dabei um einen monoklonalen Antikörper handelt, der von der Maus oder vom Menschen stammt.

**5.** Verfahren nach Anspruch 4, dadurch gekennzeichnet, daß es sich dabei handelt um den Antikörper CT-M-01.

**6.** Verfahren zur Herstellung eines aktiven Konjugats, dadurch gekennzeichnet, daß man ein Steuerungsmittel nach einem der Ansprüche 1 bis 5 über ein kovalentes Bindeglied an eine Substanz mit pharmakologischer Aktivität oder an eine Substanz, die für diagnostische Zwecke in Tumorzellen verwendbar ist, kuppelt.

**7.** Verfahren nach Anspruch 6, dadurch gekennzeichnet, daß das kovalente Bindeglied in der Praxis nur in den Lysosomen hydrolysiert wird.

**8.** Verfahren nach Anspruch 7, dadurch gekennzeichnet, daß die Hydrolyse eine enzymatische Säurehydrolyse ist.

**9.** Verfahren nach einem der Ansprüche 6 bis 8, dadurch gekennzeichnet, daß die Substanz (Komponente) ein Antikrebs-Arzneimittel ist.

**10.** Verfahren nach Anspruch 9, dadurch gekennzeichnet, daß das Antikrebs-Arzneimittel ausgewählt wird aus der Familie der Derivate von Anthracyclinen oder Vinca-Alkaloiden.

**11.** Verfahren nach Anspruch 10, dadurch gekennzeichnet, daß die Substanz (Komponente) ausgewählt wird aus Vindesin, Daunorubicin und Doxorubicin.

**12.** Verfahren nach einem der Ansprüche 6 bis 11, dadurch gekennzeichnet, daR das kovalente Bindeglied ein Hemisuccinyl-Zweig oder ein Tetrapeptid-Zweig ist.

**13.** Verfahren nach einem der Ansprüche 6 bis 12, dadurch gekennzeichnet, daß die aktive Substanz (Komponente) ausgewählt wird aus Vindesin, Daunorubicin und Doxorubicin und daß das Steuerungsmittel der Antikörper CT-M-01 ist.